# EUROPEAN PATENT APPLICATION

(11) **EP 2 172 173 A1**
(43) Date of publication of application: **07.04.2010**
(21) Application number: 08778092.0
(22) Date of filing: 11.07.2008
(51) Int. Cl.: A61F 13/56, A61F 13/15

(54) **ABSORBENT ARTICLE**

(30) Priority: 25.07.2007 JP 2007193208
(71) Applicant: Unicharm Corporation, Ehime 799-0111 (JP)
(72) Inventor: HAYASHI, Toshihisa, Kanonji-shi Kagawa 769-1602 (JP); TANIO, Toshiyuki, Kanonji-shi Kagawa 769-1602 (JP)
(74) Representative: Fitchett, Stuart Paul
(86) International application number: PCT/JP2008/062577
(87) International publication number: WO 2009/014018

(57) **Abstract**

An absorbent article that avoids damaging of adherend fibers through regulation of attaching strength. The absorbent article comprises a surface sheet (2) having liquid permeability at at least a part thereof, a liquid impermeable backside sheet (3), a liquid retentive absorbent (4) interposed between the surface sheet and the backside sheet and an attaching part (10) disposed on the external face of the backside sheet. The attaching part (10) includes a first sticky portion (11) consisting of a sticky member and a second sticky portion (12) consisting of the same sticky member as in the first sticky portion (11). The attaching part (10) is provided within an approximately rectangular area with four corners. The first sticky portion (11) is provided in the region of the outer edge of the attaching part (10) including at least the four corners. The basis weight of the sticky member in the first sticky portion (11) is greater than that in the second sticky portion (12).

## Description

### TECHNICAL FIELD

The present invention relates to an absorbent article, and in particular, to an absorbent article which is attached to an adherend, such as underwear.

### BACKGROUND ART

Sanitary napkins, panty liners, urine-absorbing pads and the like have conventionally been used as absorbent articles for absorbing excrement such as bodily fluid. These absorbent articles have: an absorbent core for absorbing and retaining bodily fluid and the like; a liquid permeable top sheet that covers the surface on a skin contacting side of the absorbent core, and a liquid impermeable back surface sheet that covers the skin noncontacting side of the absorbent core positioned on the clothing side.

These absorbent articles generally have a fastening portion composed of an adhesive material and the like on the skin noncontacting side thereof, so as to absorb excrement on the skin contacting side, for fixing the absorbent article to a crotch portion of the underwear.

However, the crotch portion of the underwear is subjected to strong deforming forces due to motion of the wearer, especially motion of wearer's legs. Therefore, the fastening portion of the absorbent article attached to the crotch portion is heavily deformed along with deformation of the underwear. This causes deformation, such as dislocation, detachment, and twisting of the absorbent article attached to the underwear, and thus produces discomfort during wearing.

The fastening portion is often formed by application of an adhesive to a rectangular region. An exterior edge and four corners of the fastening portion are subjected to more external force than a central portion thereof, and can be easily dislocated, detached, and twisted. Heavy application of an adhesive, to avoid detachment of the exterior edge and the four corners of the fastening portion, may make the absorbent article hard to remove from an adherend, such as the underwear, and may leave adhesive residue on the underwear.

An absorbent article is disclosed having adhesives of different adhesive force on a skin noncontacting side thereof as the fastening portion. An adhesive of higher adhesive force is applied to portions subjected to external forces, such as the edges of a fastening portion. An adhesive of lower adhesive force is applied to portions between the portions to which the adhesive of higher adhesive force is applied (for example, as disclosed in Japanese Unexamined Patent Application, First Publication No. 2006-296974, hereinafter referred to as Patent Document 1).

However, the absorbent article disclosed in Patent Document 1 aims at reducing noise upon removal of the absorbent article from the adherend. Applying adhesives of different adhesive force separately for this purpose requires a more complex manufacturing procedure. In addition, in a case where adhesives of different adhesive force are used, the adhesive of higher adhesive force may increase damage to fiber of the underwear.

### DISCLOSURE OF THE INVENTION

### Problems to be Solved by the Invention

The objective of the present invention is to provide an absorbent article, which has a fastening portion with adjusted fastening force.

### Means for Solving the Problems

In a first aspect of the present invention, an absorbent article is provided that includes a top sheet which is at least partially liquid permeable; a liquid impermeable back surface sheet; a liquid retentive absorbent core which is disposed between the top sheet and the back surface sheet; and a fastening portion which is disposed on an outer surface of the back surface sheet, in which: the fastening portion includes: a first adhesive portion which is composed of an adhesive material; and a second adhesive portion which is composed of an adhesive material of the same constituents as the first adhesive portion ; the fastening portion is formed in a substantially rectangular region having four corners; the first adhesive portion is formed at least in a region at an exterior edge of the fastening portion, which includes the four corners; and a basis weight of the adhesive material in the first adhesive portion is higher than a basis weight of the adhesive material in the second adhesive portion.

The fastening force of the fastening portion is adjusted by changing the basis weight of the adhesive portion formed on the fastening portion. This is because the adhesive portions of different basis weight have different degrees of adherence to a surface of an adherend such as underwear in contact therewith, thus changing the peel strength of the adhesive portion.

According to a second aspect of the present invention, in the absorbent article as described in the first aspect, the adhesive material is a room-temperature, pressure-sensitive hot melt adhesive.

According to a third aspect of the present invention, in the absorbent article as described in the first or the second aspect, the basis weight of the adhesive material in the first adhesive portion is at least 1.3 times greater than the basis weight of the adhesive material in the second adhesive portion.

According to a fourth aspect of the present invention, in the absorbent article as described in any one of the first to third aspects, a ratio between the surface area of the first adhesive portion and the surface area of the second adhesive portion is in the range of 1:1 to 1:9.

According to a fifth aspect of the present invention, in the absorbent article as described in any one of the first to fourth aspects, a peel strength of the first absorbent portion is in a range of 2.0 to 10.0 N/cm², and a peel strength of the second absorbent portion is in a range of 0.1 to 2.5 N/cm².

### Effects of the Invention

The present invention can provide an absorbent article which can avoid damaging the adherend by adjusting the fastening force of a fastening portion.

### BRIEF DESCRIPTION OF THE DRAWINGS

FIG. 1 is a top view of a panty liner according to a first embodiment of the present invention;
FIG. 2 is a cross-sectional view taken along the line Z-Z in FIG. 1;
FIG. 3 is a drawing illustrating a force applied to the panty liner;
FIG. 4 is a top view of a different example of a panty liner according to a first embodiment of the present invention;
FIG. 5 is a top view of another different example of a panty liner according to a first embodiment of the present invention; and
FIG. 6 is a top view of yet another example of a panty liner according to a first embodiment of the present invention.

### PREFERRED MODE FOR CARRYING OUT THE INVENTION

Embodiments of the present invention are described below with reference to the accompanying drawings. However, it is to be understood that the embodiments of the present invention are not limited to the following, and the technical scope of the present invention is not limited thereto.

Although the absorbent articles according to the present invention are worn on the crotch of the human body in order to absorb menstrual blood, urine, and leukorrhea discharged from the human body, the following embodiments are directed to panty liners, the primary object of which is to absorb leukorrhea discharged from the vaginal opening of females. In the following description, one of two surfaces of the absorbent article, which is directed to the excretory part, is called a "skin contacting side", and the other is called a "skin noncontacting side", irrespective of whether clothing is worn.

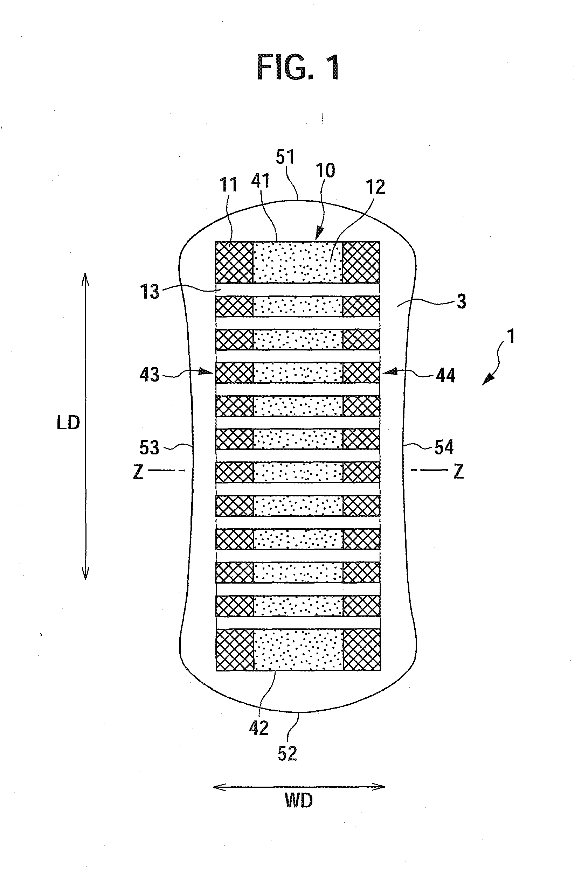

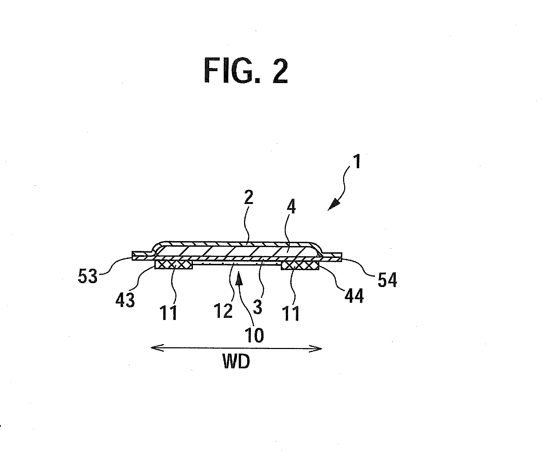

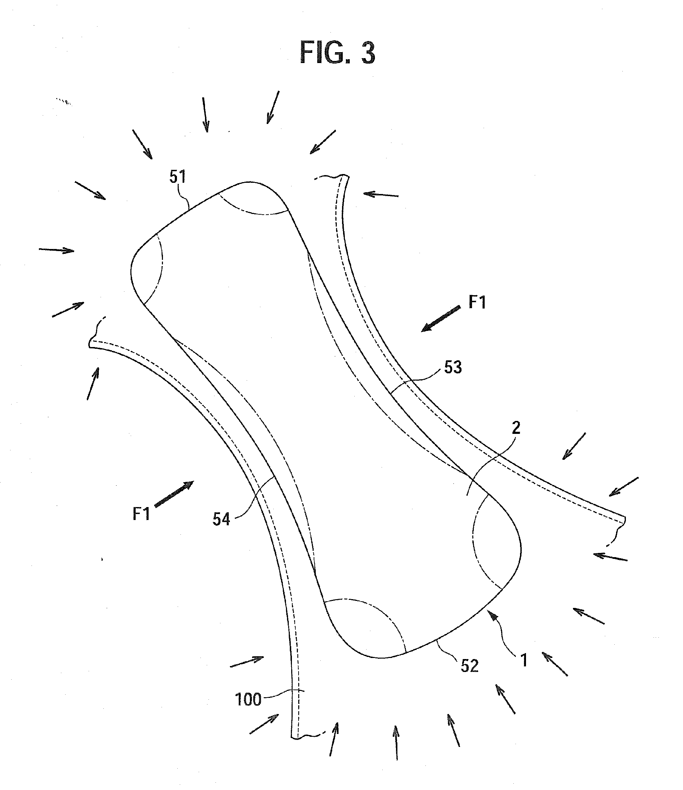

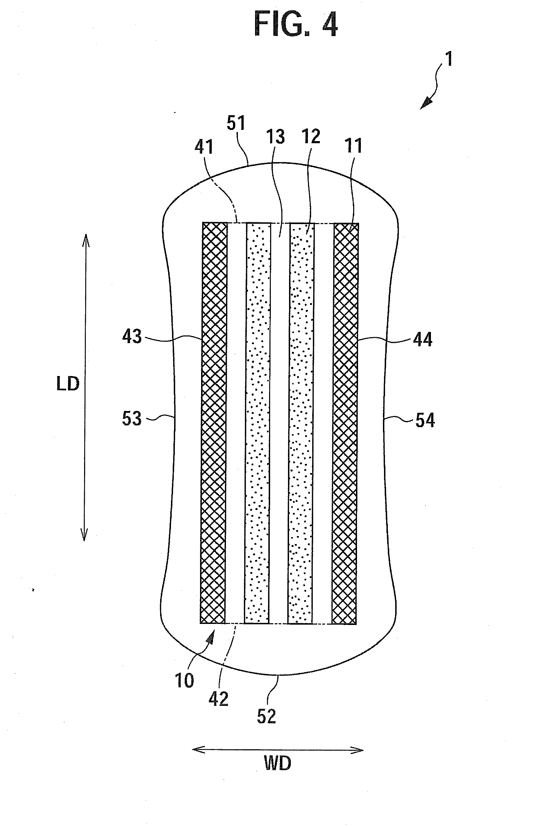

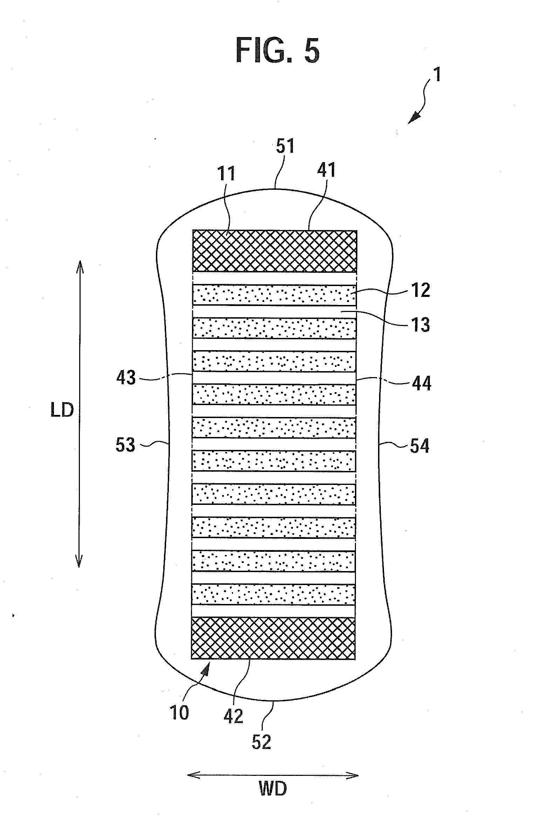

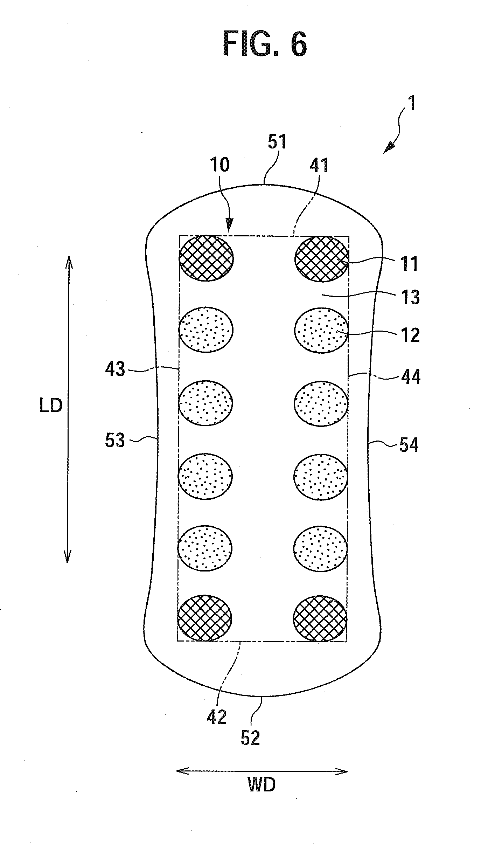

FIG. 1 is a top view of a panty liner according to a first embodiment of the present invention. FIG. 2 is a cross-sectional view taken along the line Z-Z in FIG. 1. FIG. 3 is a drawing illustrating an external force applied to the panty liner. FIG. 4 is a top view illustrating an example of the first and second adhesive portions arranged separately, along a longitudinal direction. FIG. 5 is a top view illustrating an example of the first and second adhesive portions arranged separately, along a width direction. FIG. 6 is a top view illustrating an example of the first and second adhesive portions arranged discontinuously, along the longitudinal direction.

### 1. First Embodiment

The overall configuration of the absorbent article of the present invention is described with reference to a panty liner 1 according to a first embodiment of the present invention. 1.1 General View

As shown in FIG. 1, the panty liner 1 is formed to be vertically long. The panty liner 1 has a substantially rectangular or oval shape having lengths in a longitudinal direction LD and a width direction WD. FIG. 1 shows a skin noncontacting side of the panty liner, having a fastening portion 10, which is elongated to the longitudinal direction LD of the panty liner 1, and fastens the panty liner 1 to underwear. The fastening portion 10 is formed in a substantially rectangular region having four corners. The fastening portion 10 includes adhesive portions 11 and 12 and a portion 13 of a sheet in a skin noncontacting side, between the adhesive portions, to which an adhesive material is not applied. The adhesive portion has a first adhesive portion 11 and a second adhesive portion 12, in which the basis weight of the first adhesive portion 11 is greater than that of the second adhesive portion 12. The first adhesive portion 11 is formed at least in a region at an exterior edge of the fastening portion 10, which includes the four corners. The first adhesive portion 11 projects more to the thickness direction of the panty liner 1 from the surface of the back surface sheet, than the second adhesive portion 12. The fastening force of the first adhesive portion and the second adhesive portion can thus be differentiated by changing the basis weight of the adhesive portions formed on the fastening portion 10.

The length of the panty liner 1 in a longitudinal direction LD is, for example, in the range of 100 to 200 mm, and preferably in the range of 120 to 180 mm. The length thereof in a width direction is, for example, preferably in the range of 30 to 100 mm, and more preferably in the range of 40 to 70 mm. The thickness of the panty liner 1, from the top sheet 2 to the surface of the first adhesive portion, is preferably in the range of 0.5 to 3.0 mm.

As shown in FIG. 2, the panty liner 1 includes: a liquid retentive absorbent core 4; a liquid permeable top sheet 2 disposed on a skin contacting side of the absorbent core 4, which is one side thereof in the thickness direction; and a liquid impermeable back surface sheet 3 disposed on a skin noncontacting side of the absorbent core 4, which is another side thereof in the thickness direction. The fastening portion 10 is disposed on the back surface sheet 3. The fastening portion 10 includes the first adhesive portion 11, the second adhesive portion 12, and a portion 13 of a back surface sheet 3 between one adhesive portion and the other, to which an adhesive material is not applied. The first adhesive portion 11 is formed to be continuous or discontinuous in the longitudinal direction, along an exterior edge of the fastening portion 10. The first adhesive portion 11 is formed outwardly in the width direction WD of the panty liner 1. The second adhesive portion 12 is formed between one first adhesive portion 11 on a first side edge 43 side, and another first adhesive portion 11 on a second side edge 44 side. In other words, the adhesive portion 12 is formed interiorly in the width direction WD of the panty liner 1. Here, "outwardly in the width direction" indicates toward the exterior edge of the panty liner 1, and "interiorly in the width direction" indicates toward the center in the width direction of the panty liner 1. An intermediate sheet can be disposed accordingly between the top sheet 2, the absorbent core 4, and the back surface sheet 3.

Discharged matter such as leukorrhea discharged from the excretory part of the wearer passes through the top sheet 2 and is then absorbed by the absorbent core 4. Since the back sheet 5 disposed on the skin noncontacting side is liquid impermeable, the discharged matter is absorbed by the absorbent core 4 and retained therein, without reaching the skin noncontacting surface.

### 1.2 Top sheet

The top sheet 2 is, when being worn, disposed on the wearer's body side, and also brought into contact with the excretory portion. The top sheet 2 may be entirely or partly liquid permeable, and may be composed of either a single sheet-like member or a plurality of sheet-like members bonded together. In the present embodiment, a non-woven fabric of olefin synthetic fibers, having a basis weight of 35 g/m², is used. A non-woven fabric with or without pores or a porous plastic sheet, preferably having a basis weight in the range of 10 to 100 g/m², is used as the top sheet 2.

### 1.3 Absorbent core

The absorbent core 4 absorbs and retains the discharged matter such as leukorrhea passed through the top sheet 2. A non-woven fabric with or without pores, a porous plastic sheet, and pulverized pulp, for example, is used as the absorbent core 4. The pulverized pulp can contain a superabsorbency polymer. In the present embodiment, an absorbent core having a basis weight of 25 g/m², made of rayon and polyethylene terephthalate in the ratio of 9:1, is used as the absorbent core 4.

### 1.4 Back surface sheet

The back surface sheet 3 is configured of a liquid impermeable sheet member and prevents the discharged matter and the like retained in the absorbent core 4 from leaking to a skin noncontacting side of the absorbent article. For example, a liquid impermeable polyethylene sheet having a basis weight of 22.5 g/m² is used as the back surface sheet 3.

### 1.5 Fastening portion

The fastening portion 10 is formed on an outer surface of a skin noncontacting side of the back surface sheet 3, in a substantially rectangular shape elongated in the longitudinal direction of the panty liner 1, having a longitudinal direction LD and a width direction WD. The fastening portion 10 includes a first adhesive portion 11 configured of an adhesive material, a second adhesive portion 12 having the same adhesive force as the first adhesive portion 11, and a portion 13 of a back surface sheet 3 between the adhesive portions, in which an adhesive portion is not formed. Arrangement of the first adhesive portion 11 and the second adhesive portion 12 defines the exterior edge of the fastening portion 10. The fastening portion 10 fastens the panty liner 1 to an adherend 100.

### 1.5.1 Adhesive portion

As shown in FIG. 1, the first adhesive portion 11 is formed in an exterior edge of the fastening portion 10. The second adhesive portion 12 is formed in a portion of the fastening portion 10 in which the first adhesive portion 11 is not formed, for example, between one first adhesive portion 11 and another first adhesive portion 11. The first adhesive portion 11 and the second adhesive portion 12 are composed of the same adhesive material. The same adhesive material has the same constituents. The first adhesive portion 11 and the second adhesive portion 12, therefore, have the same adhesive force. The first adhesive portion 11 is located in a position subjected to a force applied to a crotch portion of the adherend 100 due to motion of wearer's legs. In the present embodiment, the first adhesive portion 11 is formed to be continuous or discontinuous, along an exterior edge of the fastening portion 10. As described above, the fastening portion 10 includes a portion of the back surface sheet, in which an adhesive portion is not formed, between one adhesive portion and another adhesive portion. Therefore, the first adhesive portion can be formed to be continuous or discontinuous with the portion of the back surface sheet.

The fastening portion 10 is formed in a substantially rectangular shape, and the first adhesive portion is arranged at least in four corners of the fastening portion. The "substantially rectangular shape" includes a curved rectangular shape and a rectangular shape having curved sides swelling inwardly or outwardly. By arranging the first adhesive portion at least in four corners of the fastening portion, dislocation of the panty liner 1, partial detachment of the fastening portion, and twisting of the panty liner 1 can be avoided, even in a case where a crotch portion of the adherend 100 and the panty liner fastened thereto are subjected to a deforming force.

As shown in FIG. 2, a basis weight of the adhesive material in the first adhesive portion 11 is higher than a basis weight of the adhesive material in the second adhesive portion 12. The basis weight of the adhesive material in the first adhesive portion 11 is preferably at least 1.3 times greater than the basis weight of the adhesive material in the second adhesive portion 12.

For example, the basis weight of the adhesive material in the first adhesive portion 11 is preferably in the range of 25 to 100 g/m². The basis weight of the adhesive material in the second adhesive portion 12 is preferably in the range of 1 to 40 g/m².

If the basis weight of the adhesive material in the first adhesive portion 11 is lower than that of the adhesive material in the second adhesive portion 12, sufficient fastening force may not be obtained for avoiding dislocation of the panty liner 1 from the adherend 100, detachment of the fastening portion 10 from the adherend 100, and twisting of the panty liner 1.

As described above, the first adhesive portion 11 and the second adhesive portion 12 are formed so that the adhesive materials thereof have different basis weight, thus the first adhesive portion 11 projects more to the thickness direction of the panty liner 1 from the surface of the back surface sheet, than to the second adhesive portion 12. A length of the first adhesive portion 11 in a thickness direction is greater than a length of the second adhesive portion 12 in the thickness direction. In other words, the first adhesive portion 11 is thicker than the second adhesive portion 12. The first adhesive portion 11 is preferably applied so as to have a length in the thickness direction in the range of 0.02 to 0.1 mm. The second adhesive portion 12 is preferably applied so as to have a length in the thickness direction in the range of 0.001 to 0.04 mm.

If the thickness of the first adhesive portion 11 is smaller than 0.02 mm, sufficient fastening force cannot be obtained for avoiding dislocation of the panty liner 1 from the adherend 100, detachment of the fastening portion 10 from the adherend 100, and twisting of the panty liner 1. If the thickness of the first adhesive portion 11 is greater than 0.1 mm, fibers of the adherend 100 may be damaged due to the excessive fastening force.

If the thickness of the second adhesive portion 12 is smaller than 0.001 mm, sufficient fastening force cannot be obtained for supporting the adhesive force of the first adhesive portion 11, and dislocation of the panty liner 1 from the adherend 100, detachment of the fastening portion 10 from the adherend 100, and twisting of the panty liner 1 may thus occur. If the thickness of the second adhesive portion 12 is greater than 0.04 mm, fibers of the adherend 100 may be damaged due to the excessive fastening force.

The thickness of the first adhesive portion 11 is preferably at least 1.3 times greater than that of the second adhesive portion 12. A difference in thickness between the first adhesive portion and the second adhesive portion smaller than 1.3 times is not sufficient. In a case where the difference is insufficient and the basis weight of the first or the second adhesive portion is smaller than 1 g/m², the adhesive portions cannot have sufficient peel strength and lose the ability to adhere to the adherend 100. On the contrary, in a case where the difference is insufficient and the basis weight of the first or the second adhesive portion is greater than 100 g/m², the panty liner 1 adheres completely to the adherend 100 and may damage fibers thereof.

In addition, the length of the second adhesive portion 12 in the thickness direction is preferably smaller than the fiber diameter of the fiber on the surface of the adherend 100 to which the second adhesive portion 12 adheres. In this case, the first adhesive portion 11 can penetrate into the adherend 100 and fasten the panty liner 1 thereto, and the second adhesive portion 12 can immobilize the adherend 100. It is not preferable that the length of the second adhesive portion 12 in the thickness direction is greater than fiber diameter of the fiber on the surface of the adherend 100 to which the second adhesive portion 12 adheres, because both the first adhesive portion and the second adhesive portion will penetrate into the adherend 100.

The surface area of the second adhesive portion 12 is preferably greater than that of the first adhesive portion 11. More specifically, a ratio between the surface area of the first adhesive portion 11 and the surface area of the second adhesive portion 12 is 1:1 to 1:9. If the surface area of the first adhesive portion 11 is relatively smaller than that of the second adhesive portion 12, a risk of damaging the adherend 100 and of breaking the back surface sheet 3, upon removing the panty liner 1, can be reduced; however, the force for fastening the panty liner 1 will not be sufficient. On the contrary, if the surface area of the first adhesive portion 11 is relatively greater than that of the second adhesive portion 12, the fastening force is improved; however, the damage to the adherend 100 and the back surface sheet 3 increases. Based on this perspective, the ratio of surface area is preferably in the abovementioned range.

As described above, the first adhesive portion 11 is thicker than the second adhesive portion 12. Therefore, peel strength, the strength against a peeling force, of the first adhesive portion 11 is greater than peel strength of the second adhesive portion 12. More specifically, the peel strength of the first adhesive portion is preferably in the range of 2.0 to 10.0 N/cm². More preferably, the peel strength is in the range of 2.5 to 5.0 N/cm². The peel strength in the abovementioned range can reliably fasten the panty liner 1 to the adherend 100.

The peel strength of the second adhesive portion 12 is not greater than the peel strength of the first adhesive portion 11. More specifically, the peel strength of the second adhesive portion is preferably in the range of 0.1 to 2.5 N/cm². More preferably, the peel strength of the second adhesive portion is in the range of 0.5 to 2.5 N/cm². The peel strength in the abovementioned range can alleviate the problem of adhesive residue on the adherend 100 and difficulty in removal, even in a case where the panty liner 1 is firmly fastened to the adherend 100.

The peel strength is measured by a probe tack test. The test is conducted using a test apparatus such as PICMA TACKING TESTER manufactured by Imoto Machinery Co., Ltd. First, a test piece is obtained by applying an adhesive material so as to obtain a band 20 mm wide on a polyethylene film of 25 µm in thickness. The test piece is inserted into the test apparatus. A cotton cloth (JISL0803-1998, cotton shirting KANAKIN #3) cut into a 10 mm x 10 mm (1 cm²) piece, as the adherend 100, is adhered to a test probe. The test is conducted with a load of 350 g/cm², contact time of 600 seconds, and peeling speed of 30 mm/min. The measurement is performed 5 times under the condition of 23°C and 65% RH, and an average value is obtained.

In the present embodiment, a hot melt adhesive, which can be easily applied in an arbitrary pattern, is used as an adhesive material constituting the adhesive portions. The hot melt adhesive is composed of a styrene polymer, adhesive-imparting agent, and plasticizer. The styrene polymer includes, for example, a styrene-ethylene-butylene-styrene block copolymer, styrene-butadiene-styrene block copolymer, and styrene-isobutylene-styrene block copolymer. In the present embodiment, a styrene-ethylene-butylene-styrene block copolymer is used. The adhesive material is not limited thereto. Any room-temperature, pressure-sensitive hot melt adhesive can be used, which is soft and can penetrate and adhere between the fibers of the adherend 100 at room temperature. Here, "room-temperature" is 20 ±15°C. i,e, 5 to 35°C. "Room-temperature, pressure-sensitive" indicates that the adhesive can adhere to an adherend by way of a slight pressure for a short time at room temperature.

The first adhesive portion 11 and the second adhesive portion 12 are composed of the same adhesive material and have the same adhesive force per unit area. The adhesive materials are thus composed of the same constituents. The adhesive materials of the first adhesive portion and the second adhesive portion are formed to have the same adhesive force and different basis weight. In other words, the first adhesive portion is thicker than the second adhesive portion. In addition, the first adhesive portion, which is formed to be thicker, has greater peel strength than the second adhesive portion. By making the difference in thickness of adhesive portion without changing the adhesive force of the adhesive material, the first adhesive portion has greater peel strength and greater fastening force to the adherend 100.

The adherend 100 is, according to the present embodiment, female underwear (panty), which is a fabric woven from fibers. The first adhesive portion 11 and the second adhesive portion 12 of the panty liner 1 are, as described above, composed of a room-temperature, pressure-sensitive hot melt adhesive. The first and the second adhesive portions are, therefore, soft and can penetrate a gap in the structure of the adherend. In other words, when the first adhesive portion 11 and the second adhesive portion 12 are in contact with a fabric as an adherend 100, the adhesive material of the first adhesive portion 11, having a greater basis weight, penetrates between fibers of the adherend 100. In addition, the first adhesive portion, having greater thickness, penetrates between fibers of the adherend 100 and can fasten the panty liner 1 more strongly than the second adhesive portion. On the contrary, the second adhesive portion, having a smaller basis weight than the first adhesive portion, can immobilize the panty liner without damaging fibers of the adherend 100.

FIG. 3 is a drawing illustrating portions that are easily deformed, when subjected to an external force, of the panty liner 1 fastened to the adherend 100. The panty liner 1 is subjected to a force from the thigh of a wearer of the adherend 100, in a direction of an arrow F1, in other words, a force inward from outside in the width direction of the panty liner. This makes side edge portions 53 and 54 in the longitudinal direction of the panty liner 1 easy to be detached and folded upward.

In addition, a skin contacting side of the panty liner 1 is in contact with the wearer's skin, along with the adherend 100, which is dislocated and in contact with wearer's skin. In this case, four corners of the panty liner 1 are more subjected to a force from a plurality of directions (small arrows in FIG. 3) and more easily detached and folded.

For the resistance against the abovementioned external force, the adhesive portions of different peel strength are provided in the fastening portion of the panty liner. The first adhesive portion having the greater fastening force is arranged in the portions in the panty liner that are easily dislocated, detached, and twisted along with deformation of a crotch portion of the adherend 100. This can effectively inhibit the dislocation, detachment, and twisting of the panty liner. On the other hand, arranging the second adhesive portion, having the lower peel strength than the first adhesive portion, can prevent fibers of the adherend 100 from being damaged and adhesive residue from being left on the adherend 100 after removing the panty liner.

In addition, by arranging the first adhesive portion, having the greater peel strength than the second adhesive portion, in a fixed surface area, a high fixing force can be obtained without providing a large fastening portion. The fastening portion includes a part of a sheet in the back surface sheet 3 side, in which the adhesive material is not applied. This can provide superior air permeability in a case where the sheet in the back surface sheet 3 side is an air permeable sheet.

### 1.5.2 Formation of adhesive portion

As described above, the first adhesive portion 11 and the second adhesive portion 12 are formed by applying the same adhesive material in different basis weights. Therefore, an adhesive material housed in the same tank is used by pumping up using the same pump. The adhesive material is fed, under control of the same module, to a coater head as a coating member in contact with a sheet member or a skin noncontacting side of a back surface sheet, which is a target of coating.

In this case, the coater head is composed of a plurality of coater blocks. Between the plurality of coater blocks, a thin plate called a shim is inserted. The shim has a groove portion for ejecting the adhesive material. By changing the formation pattern of the groove portion, the number of shims with the groove portion to be inserted, and position of the shim, the amount of the adhesive material to be ejected from the coater head is adjusted.

In case where the shims are laminated, the portion in which one groove portion of a shim and another groove portion of another shim are laminated can eject more adhesive material than that in the portion in which the groove portion is not laminated. In this way, a basis weight of the adhesive material applied to the sheet member or the back surface sheet can be changed by changing the amount of the same adhesive material to be ejected. By changing the basis weight, the thickness of the adhesive material to be applied can also be changed.

In addition, the first adhesive portion and the second adhesive portion can be formed by so-called print coating (a method of coating an adhesive material using a general printing technique). More specifically, a concave roller, having a groove portion for forming the first adhesive portion and a shallower groove portion for forming the second adhesive portion, is rotated to transfer the adhesive material in the tank to the roller. The concave roller has groove portions of at least two different depths.

Subsequently, the adhesive material attached to the concave roller is transferred to a silicone roller rotating so as to be in contact therewith. Then, the adhesive material is transferred to a back surface sheet by rotating the silicone roller, to which the adhesive material is coated, while being in contact with a skin noncontacting side of the back surface sheet. In this step, the adhesive material of a greater basis weight from the deeper groove portion of the concave roller and the adhesive material of a lower basis weight from the shallower groove portion of the concave roller are transferred to the silicone roller. The adhesive material of a greater basis weight and the adhesive material of a lower basis weight are thus transferred to the back surface sheet. The first adhesive portion and the second adhesive portion 12 are thus formed.

### 1.6 Other configurations

Variations with different application patterns of the first adhesive portion 11 and the second adhesive portion 12 are shown in FIGS. 4 to 6. On the panty liner 1 shown in FIG. 4, the first adhesive portion 11 and the second adhesive portion 12 are formed to be continuous along the longitudinal direction LD, in the fastening portion 10. On the panty liner 1 shown in FIG. 4, the first adhesive portion 11 and the second adhesive portion 12 are formed to be separate. More specifically, in the fastening portion 10, the first adhesive portion 11 is formed outwardly in the width direction of the panty liner 1, and the second adhesive portion 12 is formed interiorly in the width direction of the panty liner 1, between two of the first adhesive portions 11. A portion 13 of a back surface sheet is present between the first adhesive portion 11 and the second adhesive portion 12. With the abovementioned configuration, a panty liner 1 can be obtained, with a superior fastening force resistant against a detaching force to a fastening portion, in the vicinity of a side edge portion in the longitudinal direction of the panty liner, due to a force from the thigh of a wearer of an adherend 100.

It should be noted that, also in a configuration where the first adhesive portion 11 and the second adhesive portion 12 are arranged adjacently, as in FIG. 1, the adhesive portions 11 and 12 can be formed along the longitudinal direction LD. In this case, the first adhesive portion 11 is formed to be adjacent to the second adhesive portion 12, and in the vicinity of both edge portions 41 and 42 of the fastening portion 10 in the longitudinal direction LD, which are arranged in the vicinity of both edge portions 51 and 52 of the panty liner 1 in the longitudinal direction LD. The second adhesive portion is formed in the fastening portion 10 interiorly in the longitudinal direction LD, between the first adhesive portions arranged in the vicinity of both of the edge portions 41 and 42 of the fastening portion 10. A portion 13 of a back surface sheet is present between a combination of the first and the second adhesive portions 11 and 12, respectively and another combination of the first and the second adhesive portions 11 and 12, respectively. With the abovementioned configuration, a panty liner 1 can be obtained, with a superior fastening force resistant against a detaching force to both of the edge portions 41 and 42 of the fastening portion, which are in the vicinity of both of the edge portions 51 and 52 in the longitudinal direction of the panty liner, due to a force applied to both of the edge portions 51 and 52 in the longitudinal direction LD of the panty liner.

FIG. 5 illustrates an example of arranging the first adhesive portion 11 and the second adhesive portion 12 separately, and arranging the first adhesive portion 11 arranged in the vicinity of both of the edge portions 41 and 42 in the longitudinal direction of the fastening portion 10. The first adhesive portion 11 and the second adhesive portion 12 are formed in parallel in the width direction, along the width direction WD of the fastening portion 10. More specifically, the first adhesive portion 11 is formed in the vicinity of both of the edge portions 41 and 42 in the longitudinal direction of the fastening portion 10, and the second adhesive portion 12 is formed interiorly in the longitudinal direction in the fastening portion 10, between two of the first adhesive portions 11. A portion 13 of a back surface sheet is present between the first adhesive portion 11 and the second adhesive portion 12. With the abovementioned configuration, a panty liner 1 can be obtained with a superior fastening force resistant against a detaching force to both of the edge portions 41 and 42 of the fastening portion, which are in the vicinity of both of the edge portions in the longitudinal direction of the panty liner, due to a force applied to both of the edge portions 51 and 52 in the longitudinal direction LD of the panty liner.

FIG. 6 is a diagram illustrating an example of arranging to be discontinuous the first adhesive portion 11 and the second adhesive portion 12, which are formed in a substantially circular shape, along the longitudinal direction of the fastening portion 10. More specifically, in the panty liner 1 shown in FIG. 6, the first adhesive portion 11 is formed in the vicinity of both of the edge portions 41 and 42 in the longitudinal direction of the fastening portion 10, and the second adhesive portion 12 is formed interiorly in the longitudinal direction of the panty liner 1, between two of the first adhesive portions 11. A portion 13 of a back surface sheet is present between the first adhesive portion 11 and the second adhesive portion 12. It should be noted that the shape of the first adhesive portion 11 and the second adhesive portion 12 is not limited to a circular shape and can be any desired shape, such as rectangle and triangle. With the abovementioned configuration, a panty liner 1 can be obtained with a superior fastening force resistant against a detaching force to both of the edge portions 41 and 42 of the fastening portion, which are in the vicinity of both of the edge portions in the longitudinal direction of the panty liner, due to a force applied to both of the edge portions 51 and 52 in the longitudinal direction LD of the panty liner. The adhesive portions 11 and 12 of the panty liner shown in FIG. 5 require less adhesive material to be applied when compared to the panty liners shown in the other diagrams, and are thus suitable for lightweight, thin absorbent articles, such as panty liners.

## Claims

1. An absorbent article comprising:
a top sheet which is at least partially liquid permeable;
a liquid impermeable back surface sheet;
a liquid retentive absorbent core which is disposed between the top sheet and the back surface sheet; and
a fastening portion which is disposed on an outer surface of the back surface sheet, wherein
the fastening portion comprises:
a first adhesive portion composed of an adhesive material; and
a second adhesive portion composed of an adhesive material of the same constituents as the first adhesive portion,
the fastening portion is formed in a substantially rectangular region having four corners,
the first adhesive portion is formed at least in a region at an exterior edge of the fastening portion, which includes the four corners and
a basis weight of the adhesive material in the first adhesive portion is higher than a basis weight of the adhesive material in the second adhesive portion.

2. The absorbent article according to claim 1,
wherein the adhesive material is a room-temperature, pressure-sensitive hot melt adhesive.

3. The absorbent article according to claim 1 or 2,
wherein the basis weight of the adhesive material in the first adhesive portion is at least 1.3 times greater than the basis weight of the adhesive material in the second adhesive portion.

4. The absorbent article according to any one of claims 1 to 3,
wherein a ratio between the surface area of the first adhesive portion and the surface area of the second adhesive portion is in the range of 1:1 to 1:9.

5. The absorbent article according to any one of claims 1 to 4,
wherein peel strength of the first adhesive portion is in a range of 2.0 to 10.0 N/cm², and peel strength of the second absorbent portion is in a range of 0.1 to 2.5 N/cm².
